# EUROPEAN PATENT SPECIFICATION

(11) **EP 2 885 047 B1**
(45) Date of publication and mention of the grant of the patent: **12.10.2016**
(21) Application number: 13744861.9
(22) Date of filing: 02.07.2013
(51) Int. Cl.: A61M 39/08, B32B 27/30, B32B 27/40, B32B 1/08

(54) **MULTI-LAYERED TUBING**
MEHRSCHICHTIGER SCHLAUCH
TUBAGE MULTICOUCHE

(30) Priority: 15.08.2012 US 201213586288; 27.11.2012 US 201213686197
(43) Date of publication of application: 24.06.2015
(60) Divisional application: 16175325.6
(73) Proprietor: Tekni-Plex, Inc., King of Prussia, PA 19406 (US)
(72) Inventor: BOURGEOIS, Philip, Perrysburg, OH 43551 (US)
(74) Representative: Jenkins, Peter David
(86) International application number: PCT/US2013/049097
(87) International publication number: WO 2014/028136

(56) References cited:
- EP-B1- 1 468 218
- WO-A1-03/064909
- DE-U1-202004 000 533
- US-A1- 2009 087 606
- US-A1- 2009 286 028

## Description

### Field of the Invention

The present invention relates to polymeric tubing typically formed by a co-extrusion process, the tubing having multiple layers of different polymeric materials.

### Background

Tubing comprised of polymeric material is used in many industrial and commercial applications, including in the medical field. Various FDA compliant plastics are used, depending upon properties desired and the intended applications. Where the tubing is used to transport fluids for in vivo treatment of human patients or for delivery of any fluid where the fluid itself or a component contained in the fluid needs to be isolated and contained within and not leach through the walls of the tube, selection of a polymeric material having a selective property can be a factor.

Polyvinyl chloride (PVC) is one of the most widely used plastics. While structurally stable and easily formable into desired shapes, PVC is typically manufactured using plasticizers which can migrate out of the PVC matrix into bodily fluids and has other properties not ideally suited for medical treatment applications. Likewise, due to the inherent nature of plasticized PVC tubing, there arises the potential absorption of medicines and other components of aqueous fluids used in medical treatments into the sidewall of the PVC tube. U.S. Pat. No. 4,627,844 to Schmitt ("Schmitt"), the disclosure of which is related to the subject matter herein, discloses a tri-layer tube which is embodied in a commercial product sold under the trademark "SUREPATH 151" by the Natvar Division of Tekni-Plex, Inc. As disclosed in Schmitt, an outer layer of PVC and an inner fluid-contact layer of low density polyethylene (LDPE) are co-extruded with an intermediate tie layer of ethylene vinyl acetate copolymer (EVA). Polyurethane is potentially a substitute for PVC as disclosed in commonly owned and co-pending U.S. Serial Nos. 13/354,029 and 13/586,288. Other tubing configurations are disclosed in U.S. Patent No. 7,647,949, U.S. Patent No. 4,211,741 and U.S. Patent Publication No. 2007/0119511, the disclosures of which are related to the subject matter herein. Examples of prior art on which the pre-characterizing portions of the independent claims are based are given in US2009286028 and US2009087606.

### Summary of the Invention

In accordance with the invention there is provided a tubing, tube or tubular device that comprises at least three concentric layers of polymeric materials comprising inner and outer layers of a polymeric material comprised of thermoplastic polyurethane and a middle layer comprised of a polymeric material comprised of an acrylate containing polymer that is disposed between and binds the inner and outer layers together by adhesion mechanisms, such as chemical adhesion, without the need for additional binders or adhesive layers. The polyurethane of which each of the inner and outer layers are comprised can be the same or different in structure, molecular weight, crystallinity, purity, monomeric unit content, branching, chain length, additive content, inorganic content and physical properties such as elongation, melting point, resistance to creep, and hardness.

The layers of polymeric materials are co-extruded together to form the tubing such that the outer and inner layers are adhered to the middle layer and thus all three layers adhered to each other. The tubing is formed with a central hollow channel, bore or passage that is radially surrounded and defined by the polymeric layers that act as the wall or walls of the tubing.

The tube or tubing is particularly suitable for transport of aqueous fluids such as fluids used in medical treatments and applications. Alternatively the tube or tubing can be used for routing, delivery and transport of liquid and gaseous non-aqueous and aqueous fluids including organic materials such as ethers, alcohols, hydrocarbons, amines, aldehydes, ketones, amides, carboxylic acids and esters and non-aqueous fluids such as oxygen, carbon dioxide, carbon monoxide, helium, neon, argon, krypton, xenon, radon, hydrogen, nitrogen and the like and mixtures of two or more of any of the foregoing with each other or one or more of the foregoing together with an aqueous fluid.

Preferably the polymeric material of the inner and outer layer is comprised of a polyurethane thermoplastic elastomeric material ("TPU") and the middle layer is comprised of an ethylene ethyl acrylate copolymer (EEA), ethylene methyl acrylate copolymer (EMA), an anhydride grafted ethylene methyl acrylate copolymer (AEMA), a copolymer of two or more of said acrylates or a mixture of two or more of the foregoing.

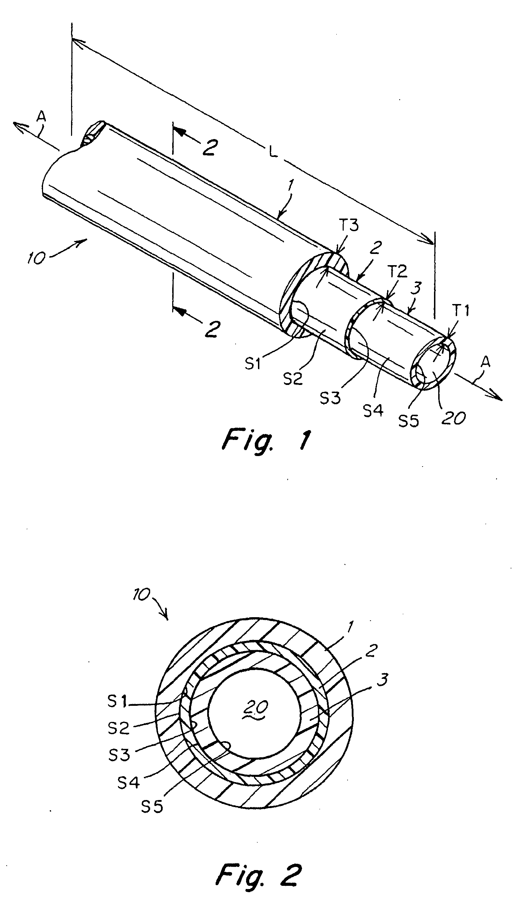

In one embodiment, the middle layer preferably has a thickness substantially greater than the combined thicknesses of the inner and outer layers. The cross-sectional thickness T2 of the middle layer, Fig. 1, typically ranges between about 5% and about 98% and more typically between about 10% and about 85% of the total cross-sectional wall thickness of the tube 10 (namely the thickness of the inner layer T1 plus the thickness of the middle layer T2 plus the thickness of the outer layer T3). The thickness T2 of the middle layer can range from about 5% to about 30% of the total cross-sectional thickness of the tube wall, or in another embodiment from about 31% to about 60%, and in yet another preferred embodiment from about 61% to about 98%. By increasing the thickness of the middle layer across the tube wall, and thus the proportional volume of the polymeric material of the middle layer of the tube vs. the volume of polymeric material of the inner and outer layers, a much lower cost and/or other benefit(s) can be obtained. For example, where the polymeric material of the middle layer is a soft ethylene ethyl acrylate (EEA) and/or ethylene methyl acrylate (EMA), these materials provide good adhesion to a thermoplastic polyurethane elastomer (TPU) and maintain resistance to delamination of the tube under high stress and temperature conditions, but are less costly than the thermoplastic polyurethane elastomers of which the inner and outer layers are comprised. Generally, the total wall thickness (T1 plus T2) of the inner and outer layers of thermoplastic polyurethane should be at least about 2 % of the total wall thickness of the tube (T1 plus T2 plus T3) for tubes greater than or equal to 2.54mm (0.100 inches) in total wall thickness, at least about 3% of the total wall thickness for tubes greater than 1.295mm (0.051 inches) in total wall thickness but below 2.54mm (0.100 inches) in total wall thickness and at least 5% of the total wall thickness for tubes greater than 0.254mm (0.010 inches) but equal to or below 1.27mm (0.050 inches) in total wall thickness in order to provide the necessary softness and elasticity along with mechanical properties required in a typical tubing application.

Thus, it is envisioned that in one embodiment the majority of the volume of the sidewall of a tube will be made from EEA or EMA material and the TPU inner layer and outer layer will be as thin as possible so as to still function as both the fluid contact layer (inner layer) and as the outer layer to which various fitments may be bonded (luers, etc.).

With reference to Figs. 1, 2, in one embodiment a tube 10 having a length L extending along a longitudinal tube axis A has inner and outer polyurethane layers 3, 1 which are between about 0.00635mm and about 0.635mm (about.00025 and about .025 inches) in thickness, T1, T3, and a middle acrylate copolymer layer 2 of between about 0.203mm and about 3.048mm (about.008 and about 0.120 inches) in thickness, T2. The layers 1, 2, 3 collectively form a tubular wall surrounding and defining a central fluid flow passage 20.

Ethylene ethyl acrylate (EEA) copolymers, ethylene methyl acrylate (EMA) copolymers and anhydride grafted ethylene methyl acrylate (AEMA) copolymers are elastomeric in nature and have excellent visual clarity. In a typical co-extrusion process, TPU and EEA or EMA or AEMA are melt extruded through a die head to form a tubular shaped extrudate that is then cooled through conventional water baths or water vacuum tanks and which are either subsequently wound or cut into a particular length for use. The level of elasticity and softness of the EEA, EMA AEMA or copolymer thereof is controlled through the amount of ethyl acrylate or methyl acrylate comonomer utilized with ethylene in the copolymerization process. The resulting three layer tubes manufactured by such a co-extrusion process act in a monolithic manner in that they return to close to their original shape and dimensions after being strained or stretched in a tensile manner along the longitudinal axis A of the tube at a stress of up to about 55 MPa and a strain of up to about 900-950 % and without any visual delaminaton between any of the layers after being submersed in water at about 60° C for about 36 hours.

The polymeric materials are preferably "contaminant free" meaning that they do not contain more than insignificant amounts of potentially unwanted materials (typically less than about out 0.5% and preferably less than about 0.2%, by weight) and/or prevent leaching or leaking of unwanted materials such as plasticizers, catalysts, monomers, metals, salts, ions or other substances that are potentially unwanted to a human being into an aqueous solution or other fluid medium with which one or the other of the three layers may come into contact during the normal course of use of the tubing in delivering aqueous (or non-aqueous) fluid, such as insulin, chemotherapy drugs and other potentially unstable aqueous drug suspensions, to or from a human subject. The middle layer prevents delamination of the outer and inner layers from the middle layer under conditions of relatively low to moderate stress or strain. In addition, the middle layer acts as a barrier to leaching or leaking of contaminants from the outer layer to or through the inner layer into the hollow central bore or passage of the tube.

In accordance with one aspect of the invention there is provided a tube comprising an inner layer, an outer layer and a middle layer, wherein the inner layer comprises a thermoplastic polyurethane, the outer layer comprises a thermoplastic polyurethane and the middle layer comprises more than about 90% by weight of an ethylene ethyl acrylate copolymer or an ethylene methyl acrylate copolymer or an anhydride grafted ethylene methyl acrylate copolymer, a copolymer of two or more of said acrylates or a mixture of two or more of the foregoing.

The inner and outer layers preferably each comprise more than about 90% by weight of an aromatic or aliphatic polyether based polyurethane.

One or both of the inner and outer layers can comprise a polytetramethyleneglycol-based polyurethane.

The ethylene methyl acrylate copolymer typically comprises at least about 19.5 percent methyl acrylate content by weight.

The ethylene ethyl acrylate copolymer typically comprises at least about 19.5 percent ethyl acrylate content by weight.

Further in accordance with the invention there is provided a method of forming a fluid delivery tube comprising;
selecting a first material comprised of more than about 90% by weight of a thermoplastic polyurethane,
selecting a second material comprised of more than about 90% by weight of a thermoplastic polyurethane,
selecting a third material comprised of more than about 90% by weight of an ethylene ethyl acrylate copolymer or an ethylene methyl acrylate copolymer or an anhydride grafted ethylene methyl acrylate copolymer, a copolymer of two or more of said acrylates or a mixture of two or more of the foregoing,
forming a tube comprised of an inner layer of the first material, an outer layer of the second material and a middle layer of the third material,
wherein the middle layer adheres the inner and outer layers together;
wherein the tube has a central fluid flow passage surrounded by the inner, middle and outer layers.

In such a method the step of forming typically comprises co-extruding the first, second and third materials simultaneously to form the inner and outer layers having a cross-sectional thickness of between about 0.00635mm and about 0.635mm (about .00025 and about .025 inches) and to form the middle layer having a cross-sectional thickness of between about 0.203mm and about 3.048mm (about .008 inches and about 0.120 inches).

The step of forming can comprise co-extruding the first, second and third materials such that the middle layer is between about 5% and about 98% of the total cross-sectional thickness of the inner, outer and middle layers combined.

The step of forming can comprise co-extruding the first, second and third materials such that the middle layer is between about 10% and about 85% of the total cross-sectional thickness of the inner, outer and middle layers combined.

The step of forming can comprise co-extruding the first, second and third materials such that the middle layer is between about 61% and about 98% of the total cross-sectional thickness of the inner, outer and middle layers combined.

In another aspect of the invention there is provided a method of delivering an aqueous fluid to a subject comprising; selecting a tube comprising an inner layer, an outer layer and a middle layer, wherein the inner and outer layers comprise more than about 90% by weight of one or more thermoplastic polyurethanes and the middle layer comprises an ethylene ethyl acrylate copolymer or an ethylene methyl acrylate copolymer or an anhydride grafted ethylene methyl acrylate copolymer, a copolymer of two or more of said acrylates or a mixture of two or more of the foregoing; wherein the tube has a central fluid flow passage surrounded by the layers; routing an aqueous fluid through the central fluid flow passage of the tube, and, delivering the aqueous fluid routed through the central fluid flow passage into a blood vessel of the subject. In such a method the step of selecting preferably comprises co-extruding the outer, inner and middle layers to form the tube such that the intermediate layer comprises at least about 90% by weight of one or more of the acrylate copolymers.

In another embodiment of the invention, such a method enables routing and delivery of liquid and gaseous non-aqueous fluids including organic materials such as ethers, alcohols, hydrocarbons, amines, aldehydes, ketones, amides, carboxylic acids, esters and the like and fluid materials such as oxygen, carbon dioxide, carbon monoxide, helium, neon, argon, krypton, xenon, radon, hydrogen, nitrogen and the like as well as mixtures of two or more of all of the foregoing with each other or mixtures of one or more of the foregoing together with an aqueous fluid.

Most preferably, the inner, middle and outer layers of the aforementioned tubes or tubing do not visually delaminate from each other at a stress up to of about 55 MPa and a strain of up to about 900-950% when measured by pulling a length of tubing about 50.8mm (2 inches) in axial length along its axis using a Lloyd LR5K plus mechanical tester at a pull rate of about 5mm/second (12 inches/minute) at ambient environmental conditions of about 72 degrees F and about 50% relative humidity, the break point of the tubing 10 being about 57-62 MPa and about 1000-1050%.

Most preferably all of the aforementioned tubes or medical tubing do not visually delaminate after being subjected to submersion in water at 60° C for 36 hours and subsequently mechanically flattened by manual squeezing of the tube from its normal round in cross-sectional condition to a flattened or oval shape cross-sectional shape or condition.

Preferably the aforementioned tubes or medical tubing have a central axial fluid flow passage through which aqueous fluid is routed, the inner layer having a radially inner wall surface that contacts the aqueous fluid, the outer and inner layers resisting delamination from the middle layer at a stress of up to about 55 MPa and a strain of up to about 900-950%.

Most preferably the middle layer serves as a barrier against, prevents or substantially lessens migration of mobile moieties such as monomers, short chained polymers, ions, water, small organic molecules, metals, plasticizers, catalysts and the like between the outer and inner layers or from the outer layer into the inner layer or the central flow passage or from the central flow passage or inner layer into the outer layer.

In another aspect of the invention there is provided a method of delivering a non-aqueous fluid comprising;
selecting a tube comprising an inner layer, an outer layer and a middle layer, wherein the inner and outer layers comprise more than about 90% by weight of a thermoplastic polyurethane and the middle layer comprises more than about 90% by weight of an ethylene ethyl acrylate copolymer or an ethylene methyl acrylate copolymer or an anhydride grafted ethylene methyl acrylate copolymer, a copolymer of two or more of said acrylates or a mixture of two or more of the foregoing;
wherein the tube has a central fluid flow passage surrounded by the layers;
routing the non-aqueous fluid through the central fluid flow passage of the tube, and,
delivering the non-aqueous fluid routed through the central fluid flow passage to a preselected repository for receipt of the fluid.

In such a method the fluid preferably comprises a liquid or gaseous non-aqueous fluid selected from the group of ethers, alcohols, hydrocarbons, amines, aldehydes, ketones, amides, carboxylic acids, esters, oxygen, carbon dioxide, carbon monoxide, helium, neon, argon, krypton, xenon, radon, hydrogen and nitrogen and mixtures of two or more of the foregoing with each other or one or more of the foregoing together with an aqueous fluid.

The tube preferably does not visually delaminate at a stress of up to about 55 MPa and a strain of up to about 900-950%.

In all of the embodiments and aspects of the invention described herein the polyurethane polymer or material of which the inner and outer layers are separately comprised, can be the same or can be different in structure, molecular weight, crystallinity, purity, monomeric unit content, branching, chain length, inorganic content and physical properties such as elongation, melting point, resistance to creep, and hardness.

### Brief Description of the Drawings

The drawings depict one or more embodiments of the invention that are shown by way of examples of the invention wherein:
Fig. 1 is a schematic perspective view of a tri-layered tube showing the outer and middle layers broken away in order to better illustrate the construction and arrangement of the tubing;
Fig. 2 is a cross-sectional view taken along lines 2-2 of the tube 10 shown in Fig. 1.

### Detailed Description

There is shown in FIG. 1 an embodiment of a co-extruded tri-layer tubing 10 according to the invention which comprises an outer layer 1 and an inner layer 3 each separately comprised of at least about 90% by weight of a polyurethane material, typically a polytetramethyleneglycol-based polyurethane one example of which is Lubrizol TPU Pellethane 2363-90AE. The tubing or tube 10 includes a middle layer 2 comprised of an ethylene ethyl acrylate copolymer, an ethylene methyl acrylate copolymer, an anhydride grafted ethylene methyl acrylate copolymer, a copolymer of two or more of said acrylates or a mixture of two or more of these acrylate based compounds or compositions. One example of a suitable ethylene ethyl acrylate copolymer is Dow Amplify EA 103 (Ethylene Ethyl Acrylate content being about 19.5% by weight). Examples of suitable ethylene methyl acrylate copolymers are Westlake MA SP2268 (Ethylene Methyl Acrylate content being about 24% by weight), Westlake MA SP2220 (Ethylene Methyl Acrylate content being about 20% by weight). One example of a suitable anhydride grafted ethylene methyl acrylate copolymer is Westlake Tymax GA 7001 (Anhydride grafted Ethylene Methyl Acrylate).

As shown in Fig. 1 the outer layer of polyurethane 1 has a radially inner facing surface S1 that binds and adheres to a radially outer facing surface S2 of the middle acrylate copolymer layer 2. Similarly the inner layer 3 has a radially outer facing surface S4 that binds and adheres to the radially inner facing surface S3 of the middle acrylate copolymer layer 2. The middle layer 2 adheres to the outer 1 and inner 3 layers such that the layers 1 and 3 remain adhered to layer 2 and to each other when the tube 10 is subjected to a stress of up to about 55 MPa and a strain of up to about 900-950% as measured by pulling a length of tubing 10 of about 50.8mm (2 inches) in axial length L along its axis A using a Lloyd LR5K Plus mechanical tester at a pull rate of about 5mm/second (12 inches/minute) at ambient environmental conditions of about 72 degrees F and about 50% relative humidity, the break point of the tubing 10 being at about 57-62 MPa and at a strain about 1000-1050%. The layers 1, 2, 3 of such tubing 10 does not visually delaminate after being subjected to submersion in water at 60° C for 36 hours and subsequently mechanically flattened by manual squeezing of the tube from its normal round in cross-sectional condition to a flattened or oval shape cross-sectional shape or condition.

As shown in Figs. 1 and 2, the layers 1, 2, 3 are formed into structurally stable walls that surround and enclose a central hollow fluid passage 20 through which a fluid is routed and flows in an axial A direction contacting the radially inner facing surface S5 of the inner layer 3. The middle layer 2 binds and holds the inner 3 and outer 1 layers together.

The inner layer 3 provides a radially inner fluid-contact surface S5, the thickness, of the inner layer 3 typically ranging in cross-sectional thickness T1 of between about 0.00635mm and about 0.635mm (about .00025 inches and about .025 inches). The middle layer 2 has a cross-sectional thickness T2 of between about 0.203mm and about 3.048mm (about .008 inches and about .120 inches,). The outer layer 1 typically ranges in cross-sectional thickness T3 of between about 0.00635mm and about 0.635mm (about .00025 inches and about .025 inches).

The polyurethane elastomer (TPU) is typically the reaction product of a polyol and isocyanate and usually includes a combination of hard and soft segment domains. An aromatic polyether-based TPU or an aliphatic polyether-based TPU can be used such as a polytetramethyleneglycol-based polyurethane. Preferred, TPU's include the Pellethane 2363-80 AE series available from the Lubrizol Corporation, Wilmington, MA such as Lubrizol TPU Pellethane 2363-90AE.

The respective thickness of each layer of tubing 10,20 can be controlled by the extrusion tooling utilized, such as the "Tri Die" extrusion apparatus manufactured by the Genca Division of General Cable Company, Clearwater, Fla. The extrusion apparatus is selected so as to provide a uniform thickness of the layers 1, 2, 3 along the substantial entirety of the axial length L of all three layers 1, 2, 3.

The polymeric materials of which the layers 1, 2, 3 are comprised are selected so as to be visually clear or transparent and manually flexible along and around the axis A of the tubing. The polymeric materials are also selected so as to maintain the integrity of the tubing 10 (namely delamination does not occur) and its transparency or clarity after being subjected to ethylene oxide (EtO) and gamma irradiation sterilization processes.

The foregoing description is intended to illustrate and not limit the scope of the invention, those skilled in the art will realize that changes and modifications may be made thereto without departing from the the invention, all such changes and modifications falling within the scope of the claims hereof.

## Claims

1. A tube (10) having a wall comprising an inner layer (3), an outer layer (1) and a middle layer (2), wherein the inner layer comprises a thermoplastic polyurethane, the outer layer comprises a thermoplastic polyurethane and **characterised in that** the middle layer comprises more than about 90% by weight of an ethylene ethyl acrylate copolymer or an ethylene methyl acrylate copolymer or an anhydride grafted ethylene methyl acrylate copolymer, a copolymer of two or more of said acrylates or a mixture of two or more of the foregoing.

2. The tube (10) of claim 1 wherein the inner layer (3) and outer layer (1) each comprise more than about 90% by weight of an aromatic or aliphatic polyether based polyurethane.

3. The tube (10) of claim 2 wherein one or both of the inner (3) and outer (1) layers comprise a polytetramethyleneglycol-based polyurethane.

4. The tube (10) of claim 3 wherein the ethylene ethyl acrylate copolymer or ethylene methyl acrylate copolymer comprises at least about 19.5 percent by weight ethyl acrylate content or methyl acrylate content.

5. The tube (10) of claim 1 wherein the inner (3) and outer (1) layers do not visually delaminate from the middle layer, at a stress up to of about 55 MPa and a strain up to about 900-950%.

6. The tube (10) of claim 1 wherein the tube does not visually delaminate when submersed in water at 60° C for 36 hours.

7. The tube (10) of claim 1 wherein the tube has a central axial fluid flow passage through which aqueous fluid is routed, the inner layer having a radially inner wall surface that contacts the aqueous fluid, the outer (1) and inner (3) layers resisting delamination from the middle layer, at a stress of up to about 55 MPa and a strain of up to about 900-950 %.

8. The tube (10) of claim 1 wherein the middle layer (2) comprises from about 5% to about 98% of the thickness of the wall, optionally wherein the middle layer (2) comprises from about 10% to about 85% of the thickness of the wall, or alternatively wherein the middle layer (2) comprises from about 61 % to about 98% of the thickness of the wall.

9. The tube (10) of claim 1 or claim 8 wherein the thickness of the middle layer (2) is between about 0.203mm (.008 inches) and about 3.048mm (0.120 inches) and the thickness of each of the inner (3) and outer (1) layers is between about 0.00635mm.(00025 inches) and about 0.635mm (.025 inches).

10. Method of forming a fluid delivery tube (10) comprising;
selecting a first material comprised of more than about 90% by weight of a thermoplastic polyurethane,
selecting a second material comprised of more than about 90% by weight of a thermoplastic polyurethane; **characterised by**
selecting a third material comprised of more than about 90% by weight of an ethylene ethyl acrylate copolymer or an ethylene methyl acrylate copolymer or an anhydride grafted ethylene methyl acrylate copolymer, a copolymer of two or more of said acrylates or a mixture of two or more of the foregoing,
forming a tube comprised of an inner layer (3) of the first material, an outer layer (1) of the second material and a middle layer (2) of the third material,
wherein the middle layer adheres the inner and outer layers together;
wherein the tube has a central fluid flow passage surrounded by the inner, middle and outer layers.

11. The method of claim 10 wherein the step of forming comprises co-extruding the first, second and third materials simultaneously to form the inner (3) and outer (1) layers having a cross-sectional thickness of between about 0.00635mm (.00025 inches) and about 0.635mm (.025 inches) and to form the middle layer having a cross-sectional thickness of between about 0.203mm (.008 inches) and about 3.048mm (0.120 inches).

12. The method of claim 10 wherein the step of forming comprises co-extruding the first, second and third materials such that the middle layer (2) is between about 5% and about 98% of the total cross-sectional thickness of the inner (3), outer (1) and middle (2) layers combined, optionally such that the middle layer (2) is between about 10% and about 85% of the total cross-sectional thickness of the inner, outer and middle layers (3,1,2) combined, or alternatively such that the middle layer (2) is between about 61% and about 98% of the total cross-sectional thickness of the inner, outer and middle layers (3,1,2) combined.

13. Method of delivering a non-aqueous fluid comprising;
selecting a tube (10) comprising an inner layer (3), an outer layer (1) and a middle layer (2), wherein the inner and outer layers comprise more than about 90% by weight of a thermoplastic polyurethane;
wherein the tube has a central fluid flow passage surrounded by the layers;
routing the non-aqueous fluid through the central fluid flow passage of the tube, and,
delivering the non-aqueous fluid routed through the central fluid flow passage to a preselected repository for receipt of the fluid,
**characterised in that** the middle layer comprises more than about 90% by weight of an ethylene ethyl acrylate copolymer or an ethylene methyl acrylate copolymer or an anhydride grafted ethylene methyl acrylate copolymer, a copolymer of two or more of said acrylates or a mixture of two or more of the foregoing

14. The method of claim 13 wherein the fluid comprises a liquid or gaseous non-aqueous fluid selected from the group of ethers, alcohols, hydrocarbons, amines, aldehydes, ketones, amides, carboxylic acids, esters, oxygen, carbon dioxide, carbon monoxide, helium, neon, argon, krypton, xenon, radon, hydrogen and nitrogen and mixtures of two or more of the foregoing with each other or one or more of the foregoing together with an aqueous fluid.

15. A tube according to any of claims 1 to 9 wherein the inner layer (3) provides a radially inner fluid contact surface.

## Patentansprüche

1. Schlauch (10) mit einer Wand, die eine innere Schicht (3), eine äußere Schicht (1) und eine mittlere Schicht (2) umfasst, wobei die innere Schicht ein thermoplastisches Polyurethan umfasst und die äußere Schicht ein thermoplastisches Polyurethan umfasst, und **dadurch gekennzeichnet, dass** die mittlere Schicht mehr als etwa 90 Gew.-% eines Ethylen-Ethylacrylat-Copolymers oder eines Ethylen-Methylacrylat-Copolymers oder eines mit Anhydrid gepfropften Ethylen-Methylacrylat-Copolymers, eines Copolymers von zwei oder mehr der Acrylate oder eines Gemischs von zwei oder mehr der vorstehenden umfasst.

2. Schlauch (10) nach Anspruch 1, wobei die innere Schicht (3) und die äußere Schicht (1) jeweils mehr als etwa 90 Gew.-% eines auf aromatischem oder aliphatischem Polyether basierten Polyurethans umfassen.

3. Schlauch (10) nach Anspruch 2, wobei eine oder beide der inneren (3) und der äußeren (1) Schicht ein auf Polytetramethylenglykol basiertes Polyurethan umfassen.

4. Schlauch (10) nach Anspruch 3, wobei das Ethylen-Ethylacrylat-Copolymer oder das Ethylen-Methylacrylat-Copolymer mindestens etwa 19,5 Gewichtsprozent Ethylacrylat-Gehalt oder Methylacrylat-Gehalt umfasst.

5. Schlauch (10) nach Anspruch 1, wobei die innere (3) und die äußere (1) Schicht bei einer Belastung von bis zu etwa 55 MPa und einer Spannung von bis zu etwa 900-950 % nicht optisch von der mittleren Schicht delaminieren.

6. Schlauch (10) nach Anspruch 1, wobei der Schlauch nicht optisch delaminiert, wenn er in Wasser bei 60 °C für 36 Stunden eingetaucht wird.

7. Schlauch (10) nach Anspruch 1, wobei der Schlauch einen zentralen axialen Fluidströmungsdurchtritt aufweist, durch den wässriges Fluid geführt wird, wobei die innere Schicht eine radial innere Wandoberfläche aufweist, die mit dem wässrigen Fluid in Kontakt tritt, wobei die äußere (1) und die innere (3) Schicht bei einer Belastung von bis zu etwa 55 MPa und einer Spannung von bis zu etwa 900-950 % einer Delaminierung von der mittleren Schicht widerstehen.

8. Schlauch (10) nach Anspruch 1, wobei die mittlere Schicht (2) von etwa 5 % bis etwa 98 % der Dicke der Wand umfasst, gegebenenfalls wobei die mittlere Schicht (2) von etwa 10 % bis etwa 85 % der Dicke der Wand umfasst oder alternativ dazu wobei die mittlere Schicht (2) von etwa 61 % bis etwa 98 % der Dicke der Wand umfasst.

9. Schlauch (10) nach Anspruch 1 oder 8, wobei die Dicke der mittleren Schicht (2) zwischen etwa 0,203 mm (0,008 Zoll) und etwa 3,048 mm (0,120 Zoll) liegt und die Dicke von jeweils der inneren (3) und der äußeren (1) Schicht zwischen etwa 0,00635 mm (0,00025 Zoll) und etwa 0,635 mm (0,025 Zoll) liegt.

10. Verfahren zum Formen eines Fluidabgabeschlauchs (10), das Folgendes umfasst:
Auswählen eines ersten Materials, das zu mehr als etwa 90 Gew.-% aus einem thermoplastischen Polyurethan besteht,
Auswählen eines zweiten Materials, das zu mehr als etwa 90 Gew.-% aus einem thermoplastischen Polyurethan besteht; **gekennzeichnet durch**
Auswählen eines dritten Materials, das zu mehr als etwa 90 Gew.-% aus einem Ethylen-Ethylacrylat-Copolymer oder einem Ethylen-Methylacrylat-Copolymer oder einem mit Anhydrid gepfropften Ethylen-Methylacrylat-Copolymer, einem Copolymer von zwei oder mehr der Acrylate oder einem Gemisch von zwei oder mehr der vorstehenden besteht,
Formen eines Schlauchs, der aus einer inneren Schicht (3) aus dem ersten Material, einer äußeren Schicht (1) aus dem zweiten Material und einer mittleren Schicht (2) aus dem dritten Material besteht,
wobei die mittlere Schicht die innere und die äußere Schicht zusammenklebt;
wobei der Schlauch einen zentralen Fluidströmungsdurchtritt aufweist, der von der inneren, der mittleren und der äußeren Schicht umgeben ist.

11. Verfahren nach Anspruch 10, wobei der Schritt des Formens das gleichzeitige Koextrudieren des ersten, des zweiten und des dritten Materials umfasst, um die innere (3) und die äußere (1) Schicht mit einer Querschnittsdicke zwischen etwa 0,00635 mm (0,00025 Zoll) und etwa 0,635 mm (0,025 Zoll) zu formen und die mittlere Schicht mit einer Querschnittsdicke zwischen etwa 0,203 mm (0,008 Zoll) und etwa 3,048 mm (0,120 Zoll) zu formen.

12. Verfahren nach Anspruch 10, wobei der Schritt des Formens das Koextrudieren des ersten, des zweiten und des dritten Materials umfasst, so dass die mittlere Schicht (2) zwischen etwa 5 % und etwa 98 % der Gesamtquerschnittsdicke der inneren (3), der äußeren (1) und der mittleren Schicht (2) kombiniert ausmacht, gegebenenfalls so dass die mittlere Schicht (2) zwischen etwa 10 % und etwa 85 % der Gesamtquerschnittsdicke der inneren, der äußeren und der mittleren Schicht (3, 1, 2) kombiniert ausmacht oder alternativ dazu so dass die mittlere Schicht (2) zwischen etwa 61 % und etwa 98 % der Gesamtquerschnittsdicke der inneren, der äußeren und der mittleren Schicht (3, 1, 2) kombiniert ausmacht.

13. Verfahren zum Abgeben eines nichtwässrigen Fluids, das Folgendes umfasst:
Auswählen eines Schlauchs (10), der eine innere Schicht (3), eine äußere Schicht (1) und eine mittlere Schicht (2) umfasst, wobei die innere und die äußere Schicht mehr als etwa 90 Gew.-% eines thermoplastischen Polyurethans umfassen;
wobei der Schlauch einen zentralen Fluidströmungsdurchtritt aufweist, der von den Schichten umgeben ist;
Führen des nichtwässrigen Fluids durch den zentralen Fluidströmungsdurchtritt des Schlauchs und
Abgeben des nichtwässrigen Fluids, das durch den zentralen Fluidströmungsdurchtritt geführt wird, an ein vorher ausgewähltes Behältnis zur Aufnahme des Fluids,
**dadurch gekennzeichnet, dass** die mittlere Schicht mehr als etwa 90 Gew.-% eines Ethylen-Ethylacrylat-Copolymers oder eines Ethylen-Methylacrylat-Copolymers oder eines mit Anhydrid gepfropften Ethylen-Methylacrylat-Copolymers, eines Copolymers von zwei oder mehr der Acrylate oder eines Gemischs von zwei oder mehr der vorstehenden umfasst.

14. Verfahren nach Anspruch 13, wobei das Fluid ein flüssiges oder gasförmiges nichtwässriges Fluid umfasst, das aus der Gruppe von Ethern, Alkoholen, Kohlenwasserstoffen, Aminen, Aldehyden, Ketonen, Amiden, Carbonsäuren, Estern, Sauerstoff, Kohlendioxid, Kohlenmonoxid, Helium, Neon, Argon, Krypton, Xenon, Radon, Wasserstoff und Stickstoff und Gemischen von zwei oder mehr der vorstehenden miteinander oder einem oder mehreren der vorstehenden zusammen mit einem wässrigen Fluid ausgewählt ist.

15. Schlauch nach einem der Ansprüche 1 bis 9, wobei die innere Schicht (3) eine radial innere Fluidkontaktoberfläche bereitstellt.

## Revendications

1. Un tube (10) ayant une paroi comprenant une couche intérieure (3), une couche extérieure (1) et une couche intermédiaire (2), dans lequel la couche intérieure comprend un polyuréthane thermoplastique, la couche extérieure comprend un polyuréthane thermoplastique, et **caractérisé en ce que** la couche intermédiaire comprend plus de environ 90 % en poids d'un copolymère d'éthylène acrylate d'éthyle ou d'un copolymère d'éthylène acrylate de méthyle ou d'un copolymère d'éthylène acrylate de méthyle greffé avec un anhydride, d'un copolymère constitué de deux ou plus de deux desdits acrylates ou d'un mélange de deux ou plus de deux des éléments précités.

2. Le tube (10) selon la revendication 1 dans lequel la couche intérieure (3) et la couche extérieure (1) comprennent chacune plus de environ 90 % en poids d'un polyuréthane à base de polyéther aromatique ou aliphatique.

3. Le tube (10) selon la revendication 2 dans lequel une ou les deux des couches intérieure (3) et extérieure (1) comprennent un polyuréthane à base de polytétraméthylène glycol.

4. Le tube (10) selon la revendication 3 dans lequel le copolymère d'éthylène acrylate d'éthyle ou le copolymère d'éthylène acrylate de méthyle comprend au moins environ 19,5 % en poids de contenu d'acrylate d'éthyle ou de contenu d'acrylate de méthyle.

5. Le tube (10) selon la revendication 1 dans lequel les couches intérieure (3) et extérieure (1) ne se délaminent pas visuellement de la couche intermédiaire, à une contrainte allant jusqu'à environ 55 MPa ni à une déformation allant jusqu'à environ 900-950 %.

6. Le tube (10) selon la revendication 1 dans lequel le tube ne se délamine pas visuellement lorsqu'il est immergé dans de l'eau à 60°C pendant 36 heures.

7. Le tube (10) selon la revendication 1 dans lequel le tube a un passage d'écoulement de fluide central axial à travers lequel le fluide aqueux est acheminé, la couche intérieure ayant une surface de paroi radialement intérieure qui est en contact avec le fluide aqueux, les couches extérieure (1) et intérieure (3) résistant à la délamination de la couche intermédiaire, à une contrainte allant jusqu'à environ 55 MPa et à une déformation allant jusqu'à environ 900-950 %.

8. Le tube (10) selon la revendication 1 dans lequel la couche intermédiaire (2) comprend entre environ 5 % et environ 98 % de l'épaisseur de la paroi, en option dans lequel la couche intermédiaire (2) comprend entre environ 10 % et environ 85 % de l'épaisseur de la paroi, ou alternativement dans lequel la couche intermédiaire (2) comprend entre environ 61 % et environ 98 % de l'épaisseur de la paroi.

9. Le tube (10) selon la revendication 1 ou la revendication 8 dans lequel l'épaisseur de la couche intermédiaire (2) est comprise entre environ 0,203 mm (0,008 pouce) et environ 3,048 mm (0,120 pouce) et l'épaisseur de chacune des couches intérieure (3) et extérieure (1) est comprise entre environ 0,00635 mm (0,00025 pouce) et environ 0,635 mm (0,025 pouce).

10. Un procédé de formation d'un tube de distribution de fluide (10) comprenant :
la sélection d'un premier matériau composé de plus de environ 90 % en poids d'un polyuréthane thermoplastique,
la sélection d'un deuxième matériau composé de plus de environ 90 % en poids d'un polyuréthane thermoplastique ; **caractérisé par**
la sélection d'un troisième matériau composé de plus de environ 90 % en poids d'un copolymère d'éthylène acrylate d'éthyle ou d'un copolymère d'éthylène acrylate de méthyle ou d'un copolymère d'éthylène acrylate de méthyle greffé avec un anhydride, d'un copolymère constitué de deux ou plus de deux desdits acrylates ou d'un mélange de deux ou plus de deux des éléments précités,
la formation d'un tube composé d'une couche intérieure (3) du premier matériau, d'une couche extérieure (1) du deuxième matériau et d'une couche intermédiaire (2) du troisième matériau,
dans lequel la couche intermédiaire fait adhérer les couches intérieure et extérieure ensemble ;
dans lequel le tube a un passage d'écoulement de fluide central entouré par les couches intérieure, intermédiaire et extérieure.

11. Le procédé selon la revendication 10 dans lequel l'étape de formation comprend la co-extrusion des premier, deuxième et troisième matériaux simultanément pour former les couches intérieure (3) et extérieure (1) ayant une épaisseur de la section transversale comprise entre environ 0,00635 mm (0,00025pouce) et environ 0,635 mm (0,025 pouce) et pour former la couche intermédiaire ayant une épaisseur de la section transversale comprise entre environ 0,203 mm (0,008 pouce) et environ 3,048 mm (0,120 pouce).

12. Le procédé selon la revendication 10 dans lequel l'étape de formation comprend la co-extrusion des premier, deuxième et troisième matériaux de telle sorte que la couche intermédiaire (2) est comprise entre environ 5 % et environ 98 % de l'épaisseur totale de la section transversale des couches intérieure (3), extérieure (1) et intermédiaire (2) combinées, en option de telle sorte que la couche intermédiaire est comprise entre environ 10 % et environ 85 % de l'épaisseur totale de la section transversale des couches intérieure, extérieure et intermédiaire (3, 1,2) combinées, ou alternativement de telle sorte que la couche intermédiaire (2) est comprise entre environ 61 % et environ 98 % de l'épaisseur totale de la section transversale des couches intérieure, extérieure et intermédiaire (3, 1,2) combinées.

13. Un procédé de distribution d'un fluide non aqueux comprenant :
la sélection d'un tube (10) comprenant une couche intérieure (3), une couche extérieure (1) et une couche intermédiaire (2), dans lequel les couches extérieure et intérieure comprennent plus de environ 90 % en poids d'un polyuréthane thermoplastique ;
dans lequel le tube a un passage d'écoulement de fluide central entouré par les couches ;
l'acheminement du fluide non aqueux à travers le passage d'écoulement de fluide central du tube ; et
la distribution du fluide non aqueux acheminé à travers le passage d'écoulement de fluide central vers un dépôt présélectionné pour la réception du fluide,
**caractérisé en ce que** la couche intermédiaire comprend plus de environ 90 % en poids d'un copolymère d'éthylène acrylate d'éthyle ou d'un copolymère d'éthylène acrylate de méthyle ou d'un copolymère d'éthylène acrylate de méthyle greffé avec un anhydride, d'un copolymère constitué de deux ou plus de deux desdits acrylates ou d'un mélange de deux ou plus de deux des éléments précités.

14. Le procédé selon la revendication 13 dans lequel le fluide comprend un fluide non aqueux liquide ou gazeux sélectionné dans le groupe des éthers, alcools, hydrocarbures, amines, aldéhydes, cétones, amides, acides carboxyliques, esters, oxygène, dioxyde de carbone, monoxyde de carbone, hélium, néon, argon, krypton, xénon, radon, hydrogène et azote et des mélanges de deux ou plus de deux des éléments précités ou d'un ou de plusieurs des éléments précités avec un fluide aqueux.

15. Un tube selon l'une quelconque des revendications 1 à 9, dans lequel la couche intérieure (3) fournit une surface de contact du fluide radialement intérieure.
